# EUROPÄISCHE PATENTANMELDUNG

(11) **EP 1 321 096 A2**
(43) Veröffentlichungstag der Anmeldung: **25.06.2003**
(21) Anmeldenummer: 02027429.6
(22) Anmeldetag: 09.12.2002
(51) Int. Cl.: A61B 5/055

(54) **Verfahren zur Bestimmung des Gesamtkörper-Fettgehaltes**

(30) Priorität: 20.12.2001 DE 10163015
(71) Anmelder: SIEMENS AKTIENGESELLSCHAFT, 80333 München (DE)
(72) Erfinder: Kuth, Rainer, 91074 Herzogenaurach (DE); Rupprecht, Thomas, Prof., 91080 Uttenreuth (DE); Wagner, Maren, Dr., 91052 Erlangen (DE)

(57) **Zusammenfassung**

Verfahren zur hochgenauen Bestimmung des Körper-Fettgehaltes, wobei der Proband mittels eines auf die Fett-Resonanzfrequenz verstimmten MR-Scanners vermessen wird.

## Beschreibung

Die Bestimmung des Körper-Fettgehaltes beim menschlichen Körper geschieht heute nahezu ausschließlich anhand der Körpermasse. Die hierbei erzielte Genauigkeit mag für eine Absolutmessung in vielen Fällen akzeptabel sein. Für eine Relativmessung beim einzelnen Patienten, wenn eine Therapie überwacht werden soll, ist diese Methode jedoch zu ungenau. Dies liegt daran, dass die Körpermasse kurzzeitig relativ großen Schwankungen unterliegt, durch Nahrungsaufnahme und -ausscheidung, Wasseraufnahme im Gewebe und den deutlich schwankenden Eiweißgehalt im Körper. So gibt es Therapien, welche als Nebeneffekt die Ausscheidung von bis zu 3 Litern Wasser in nur wenigen Stunden bewirkt und umgekehrt kann ein Mensch aber auch bis zu 6 Liter Wasser pro Tag trinken.

Die Bestimmung der Körpermasse ist für die Diagnose bei einer Reihe von Erkrankungen außerordentlich wichtig. Ein zu hoher Fettgehalt stellt beispielsweise ein hohes Risiko dar, für Diabetes sowie Gefäß- und Gelenkkrankheiten.

Der Erfindung liegt daher die Aufgabe zugrunde, ein Verfahren zu schaffen, welches eine hohe absolute und je Patient eine sehr genaue relative Genauigkeit aufweist.

Zur Lösung dieser Aufgabe ist erfindungsgemäß vorgesehen, dass der Proband mittels eines auf die Fett-Resonanzfrequenz verstimmten MR-Scanners vermessen wird, wobei sowohl eine Volumenmessung des Fettes über den ganzen Körper als auch - bevorzugt - eine integrale Messung über eine oder mehrere Schichten erfolgen kann.

Durch die erfindungsgemäße Vermessung des Fettgehaltes mithilfe eines MR-Scanners - wobei im Gegensatz zum "normalen" Einsatz eines solchen MR-Scanners - dieser nicht auf die Wasserfrequenz sondern auf die etwas abweichende Fettfrequenz abgestimmt ist, lässt sich der im Zusammenhang mit Risikoerkrankungen besonders wesentliche subkutane Fettgehalt sehr genau bestimmen.

Bei der integralen Messung zur Bestimmung des relativen Fettgehaltes können dabei die Schichten anhand anatomischer Landmarken ausgewählt werden. Anatomische Landmarken sind z. B. die Wirbelsäule für eine sagitale Messung und der Bauchnabel für eine axiale Messung.

Besonders rasch und einfach lässt sich die Fettbestimmung durchführen, wenn die Messung mit einer TrueFisp Sequenz, oder auch andere Sequenzen, erfolgt. Die Messzeit zur Gewinnung eines Schnittbildes mit einer solchen TrueFisp Sequenz beträgt nur ca. 1 Sekunde.

Wird der Proband zur schichtweisen Erfassung des gesamten Körpers durch Verschieben des Lagerungstisches durch den MR-Scanner bewegt und in den Einzelbildern die Fettschicht segmentiert und aus dem Gesamtvolumen der Fettgehalt errechnet, so bedarf es hierzu unter Zugrundelegung von Schichtdicken von ca. 2 cm einer Folge von z. B. 90 axialen Schnitten vom Kopf bis Fuß, die eine Messzeit von nur 3 Minuten erfordern.

Weitere Vorteile, Merkmale und Einzelheiten der Erfindung ergeben sich aus der nachfolgenden Beschreibung eines Ausführungsbeispiels sowie anhand der Zeichnung. Dabei zeigen:
- Fig. 1: ein Schichtbild axial bei einem normalgewichtigen Patienten,
- Fig. 2: ein Schichtbild axial bei einem übergewichtigen Patienten und
- Fig. 3: das Schichtbild nach Fig. 2, wobei das Fett segmentiert worden ist.

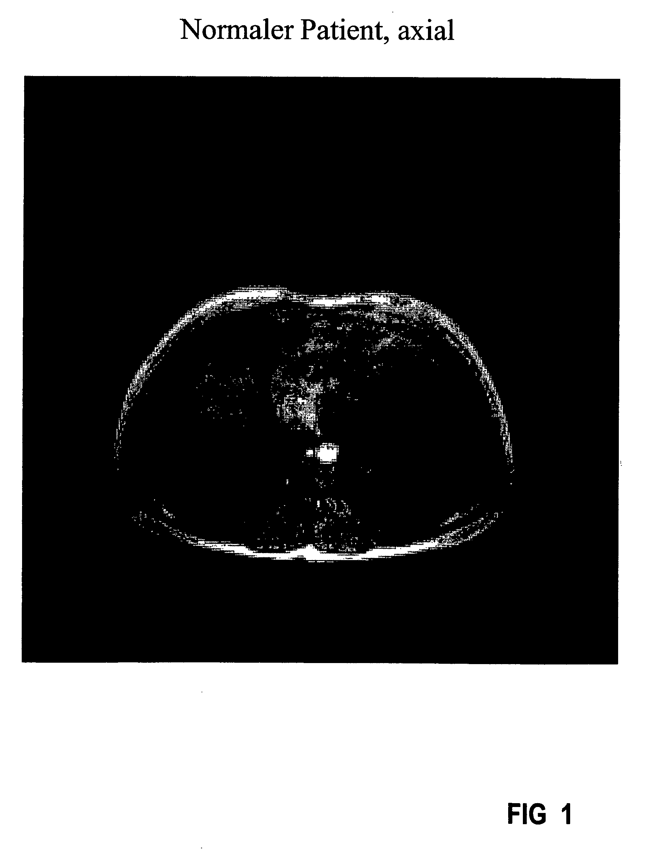

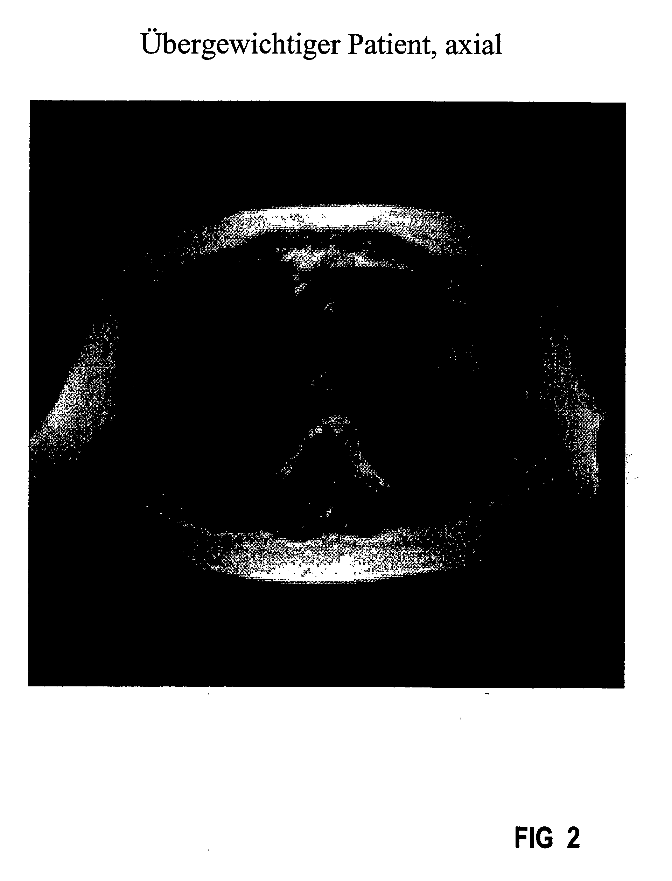

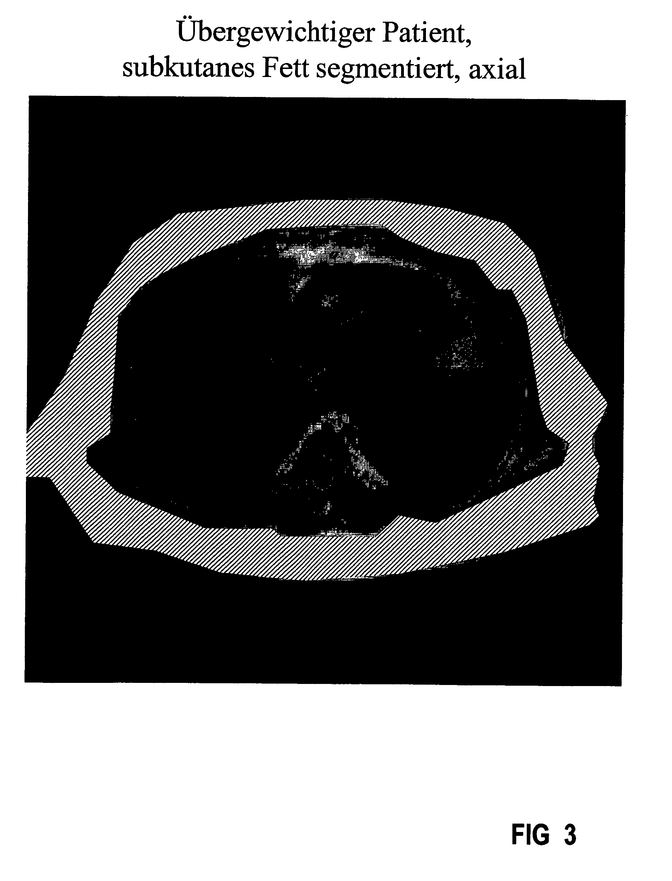

Aus dem Vergleich der beiden Bilder in den Figuren 1 und 2 kann man sehr deutlich erkennen, wie sehr die subkutane Fettschicht bei übergewichtigen Patienten gegenüber normalgewichtigen erhöht ist. Aus der Fläche dieser durch Einsatz eines auf die Fett-Resonanzfrequenz abgestimmten MR-Scanners gewonnenen Schichtbildes, was sehr einfach durch Segmentierung und Umringung der Schicht des hohen Fettgehaltes entsprechend Fig. 3 erfolgen kann, lässt sich das Fettvolumen in den einzelnen Schichten sehr genau bestimmen und aus der Addition der Fettvolumina die Gesamtfettmasse im Körper wesentlich exakter als bei allen bisher bekannten Verfahren bestimmen.

## Patentansprüche

1. Verfahren zur hochgenauen Bestimmung des Körper-Fettgehaltes, **dadurch gekennzeichnet, dass** der Proband mittels eines auf die Fett-Resonanzfrequenz verstimmten MR-Scanners vermessen wird.

2. Verfahren nach Anspruch 1, **dadurch gekennzeichnet, dass** eine Volumenmessung des Fettes über den ganzen Körper erfolgt.

3. Verfahren nach Anspruch 1, **dadurch gekennzeichnet, dass** eine integrale Messung über eine oder mehrere Schichten durchgeführt wird.

4. Verfahren nach Anspruch 3, **dadurch gekennzeichnet, dass** bei der integralen Messung zur Bestimmung des relativen Fettgehaltes die Schichten anhand anatomischer Landmarken ausgewählt werden.

5. Verfahren nach Anspruch 3 oder 4, **dadurch gekennzeichnet, dass** die Messung mit einer TrueFisp Sequenz erfolgt.

6. Verfahren nach einem der Ansprüche 3 bis 5, **dadurch gekennzeichnet, dass** der Proband zur schichtweisen Erfassung des gesamten Körpers durch Verschieben des Lagerungstisches durch den MR-Scanner bewegt und in den Einzelbildern die Fettanteile segmentiert werden und aus dem Gesamtvolumen der Gesamt-Fettgehalt des Körpers errechnet wird.
